# EUROPEAN PATENT SPECIFICATION

(11) **EP 1 545 562 B1**
(45) Date of publication and mention of the grant of the patent: **19.04.2006**
(21) Application number: 03794354.5
(22) Date of filing: 09.09.2003
(51) Int. Cl.: A61K 31/715, A61P 3/04

(54) **BRANCHED ALPHA-GLUCANS FOR WEIGHT MANAGEMENT**
VERZWEIGTE ALPHA-GLUCANE FüR DAS GEWICHTSMANAGEMENT
ALPHA-GLUCANES RAMIFIES POUR GESTION DU POIDS

(30) Priority: 09.09.2002 EP 02078684
(43) Date of publication of application: 29.06.2005
(73) Proprietor: NEDERLANDSE ORGANISATIE VOOR TOEGEPAST-NATUURWETENSCHAPPELIJK ONDERZOEK TNO, 2628 VK Delft (NL)
(72) Inventor: EKHART, Peter, Frank, NL-1078 VJ Amsterdam (NL); VAN GEEL-SCHUTTEN, Gerritdina, Hendrika, NL-3971 XG Driebergen (NL); VAN BINSBERGEN, Marie, Madelon, NL-6708 AE WAGENINGEN (NL); TIMMERMAN, Eef, Jan, NL-3931 HA Woudenberg (NL)
(74) Representative: van Westenbrugge, Andries
(86) International application number: PCT/NL2003/000625
(87) International publication number: WO 2004/022076

(56) References cited:
- EP-A- 0 506 166
- EP-A- 0 550 060
- EP-A- 0 719 783
- WO-A-82/03329
- DE-A- 19 860 375
- FR-A- 2 486 400
- US-A1- 2002 035 089

## Description

### Field of the invention

The invention is in the field of body weight management. In particular, the invention is concerned with a method for inducing satiety feeling by incorporating a satiety-inducing compound into a liquid product suitable for human consumption.

### Background of the invention

The incidence of obesity-related diseases is continuously rising among the developed populations, affecting about 250 million people world-wide with a double digit growth annually. Also incidence for overweight is increasing with a prevalence of 800 million people world-wide.

In their search for effective solutions for the global health problem of obesity among the Western population, both the pharmaceutical and the food industry have developed several concepts for inducing or extending satiety feeling for the consumer.

The pharmaceutical industry has aimed at developing targeted specific inhibitors of the digestive enzymes, fat replacers or burners. Examples of pharmaceutical active compounds affecting satiety feeling like sibutramine (Roche) and digestive enzymes like Orlistat (Knoll/BASF) or Acarbose (Bayer, US 4,062,950). Common dosage forms for these active compounds are powders and tablets.

The food industry has focused on the development of meal replacers and supplements containing satiety-inducing ingredients, reduced energy components and also burners. Examples of meal replacers marketed by food industry are Slimfast (Unilever), Profiel (Numico), Ensure (Abbott) and Modifast (Novartis). These products are presented in a solid form, for example a food bar, a powder blend, a bakery product or in liquid forms like a nutritious drink.

Burners used in food products but also supplements are for example caffeine combined with ephedrine, increasing the energy metabolism. Examples of reduced energy components used in food products are sucrose esters like Olestra, dietary fibres and reduced fat products. Dietary fibres can also induce a slow down of the stomach emptying and a slow uptake of nutrients.

The combined portfolio of commercial products offered have appeared to be little effective on the management of weight in human volunteer studies, with some exceptions in the food and pharma market. Especially for the meal replacers little proof of principle has been demonstrated (Critical reviews in food science and nutrition, (2001), **41** (1), 45-70).

Solutions for satiety inducement known in the art are for example mentioned in WO 01/30231 describing a proteinase inhibitor, claimed to induce a feed-back mechanism for CCK production. CCK is known to be related to satiation. The common food ingredients known to affect satiety and satiation can also be combined in order to gain maximum effect on satiety feeling like is done by Pacific Health Laboratories (US 6,207,638) in their Satietrol product line. The product contains protein, long chain fatty acids, calcium and soluble and insoluble fibres claimed to affect the production of cholecystokinin (CCK). US 4,833,128 describes a formula with a high phenylalanine content also claimed to stimulate CCK production by the body.

Other active compounds claimed to affect satiety feeling directly are described in WO 97/31943, (GLP-2 peptide), WO 98/20895 (GLP-1 peptide), WO 00/22937 (leptin), WO 99/55331 (α-lipoic acid), WO 95/29676 (cotinine), GB 2165452 (pseudoephedrine) and WO 01/20991 (phytochemicals).

Several carrier systems are described in order to create a targeted delivery of particular active components in the digestive tract like for example WO 01/17377 and DE 19942417 both using uronic acid containing polysaccharides optionally crosslinked as a carrier for actives, WO 93/24113 using formulas creating delayed uptake of nutrients in order to suppress hunger feeling, and US 5,753,253 employing pH sensitive coatings to deliver actives in the ileum.

Strategies aiming at inhibition of digestive enzymes have been described in US 5,726,291 (α-amylase) and lipase inhibitors (Orlistat).

Another route for inhibiting glucose uptake by the human digestive tract has been described in patent application US 2002/0037577, claiming the use of probiotic micro-organisms like *L. reuteri* in order to capture digestible sugars and to convert these into non-digestible polysaccharides. The probiotic micro-organisms are preferably dosed in enteric coated capsule designed to release the micro-organisms in the small intestine.

Finally strategies affecting the gastric emptying or delayed uptake of nutrients are described in US 4,689,219 (xanthan - locust bean gum blends), WO 00/22937 (gelled protein particles for delayed uptake of amino acids) and EP 0471217 (double gelatine layer for release of stomach filling agent).

The solutions described in the art have mainly been developed using solid dosage forms for the active compounds being either a compound directly affecting satiety feeling, an inhibitor, a slow release compound or a burner. On the other hand, liquid formulas are limited and are focussed on the delivery of particular actives or they are part of the meal replacers like the designer drinks as Slim fast or Ensure. For the liquid formulas it would be very desirable to have an active compound which can be formulated in a liquid product without affecting the preferred sensory attributes of the drink. This active compound should preferably demonstrate no excessive thickening or gelling properties in the liquid product. However if this compound could also create satiation after entering the stomach a new concept for the development of liquid weight management formulas would become available.

Well known examples of compounds claimed to have satiety-inducing effects are hydrocolloids incorporated in solid formulas, creating a significant thickening or gelling effect in the stomach. This leads to a feeling of satiety and provides possibilities to manage the delivery of nutrients or other active compounds to the ileum. Hydrocolloids used in liquid products have a low level of dosage ranging between 0.01 and 1 wt% and will not demonstrate an increase in viscosity after entering the stomach, relative to a product not containing any hydrocolloid. A theoretical exception to this rule is formed by particular low methylated pectins which are able to increase the viscosity in case the acidity is increased. In practise this concept can not be used due to the high sensitivity of this type of pectin for calcium cations, which destroys the acid effect on viscosity (Gilsenan, P.M. thermally reversible acid induced gelation of low-methoxy pectin, (1999), **41**, 339-349.

Also insoluble dietary fibres like wheat fibre or apple fibres are known to be effective in creating a saturated feeling if used in a liquid product. Disadvantage of these insoluble fibres is the limited level of dosage in liquid products due to destabilisation effects in the liquid product or undesired appearance of the liquid product. These undesired appearances include precipitation of fibre and cloudiness.

Additionally the water binding effect of dietary fibres is limited if dosed via a liquid product in contrast to solid products. Additionally other active compounds can bind irreversibly to insoluble dietary fibres reducing their nutritional efficacy.

The limitations in the art described have created the need for a polysaccharide which can be used to stabilise liquid products without the problem of undesired appearances, unwanted changes in texture, intolerance problems for the consumer, the caloric content and with the ability to increase the residence time in the stomach. The current invention provides a solution for this need as explained in the description of the invention.

### Description of tite invention

It was found that satiety feeling can be induced by incorporating into a liquid product for human consumption a highly branched α-glucan. When consumed by a person, the viscosity of the liquid product drastically increases triggered by the low pH conditions of the stomach. The method is effective when using varying levels of glucan, e.g. ranging between 1 and 10 wt% on total liquid protein-containing product, in particular in the presence of a protein. Thus, the invention pertains to the use of branched α-glucans for preparing a nutritional or pharmaceutical composition suitable for satiation or enhancing satiety in a human.

It was observed that α-glucans having a certain elevated degree of branching combined with a particular molecular weight surprisingly induce a thickening effect in case of an increase of the acidity in the product, especially in the presence of protein. This effect is favourable for the formulation of liquid products adding stabilising and texturising functionality in the drink without affecting the preferred sensory attributes and appearance. After lowering of the pH, a significant thickening effect occurs leading to a prolonged and enhanced satiation and satiety feeling in contrast to existing liquid product formulations using polysaccharides as an active compound.

The α-glucans to be used according to the invention should have a degree of branching of at least 8%. The degree of branching is defined as the total number of branching anhydroglucose units (AGU), i.e. AGU being bound to three other units, with respect to the total number of AGU of a molecule. The degree of branching can be determined by methods known in the art, like selective hydrolysis using isoamylase followed by a coulometric titration with iodine, or methylation of the polysaccharide followed by hydrolysis and gas chromatography analysis. The preferred minimum degree of branching is 10%, more preferred at least 12%, up to e.g. 24%.

In general, a branched polysaccharide consists of three types of units, (a) terminal units, being bound to one other unit only, (b) chain units, being bound to two other units, and (c) branching units, being bound to three other units. The total number of branching units is equal to the total number of terminal units minus two. Thus, in polysaccharides of a certain length (e.g. over about 1,000 units, i.e. with a molar weight over 160,000) the percentage of branching units becomes about equal to the percentage of terminal units.

As methods of determining the precise proportion of branching and terminal units may result in slightly different values from method one to another, the degree of branching can also be defined as the percentage of branching units plus the percentage of terminal units, divided by two.

The glucans to be used according to the invention contain, as a result of their branched nature, at least two types of linking one AGU to another. The type may be 1,2-linking, 1,3-linking, 1,4-linking or 1,6-linking. The glucans of the invention contain at least 1,6-linkages. Preferably the branched α-glucans contain both 1,4-linkages and 1,6-linkages, the branching units being 1,4,6-linked and amounting to at least 8% of the AGU. The glucans may also contain 1,3 and 1,6 linkages such as the mutan-type glucans described in WO 03/008618. The glucans may have an average molecular weight of at least 10⁵ Da, preferably at least 5·10⁵ Da, more preferably at least 2.5·10⁶ Da, up to e.g. 10⁹, in particular up to 10⁸ Da. The glucans are preferably non-ionic, and more preferably non-derivatised, although a degree of substitution with e.g. acyl groups, carboxyl groups, hydroxyalkyl groups up to about 5% will be acceptable.

Preferably the glucans to be used can be obtained from sucrose by glucosyl transferase activity (*de novo* synthesis), for example expressed by food approved micro-organisms like *L. reuteri* (WO 01/90372). Alternatively, the highly branched α-glucans can be derived by modification of commonly available glucans - like starch, amylopectin or glycogen - having an insufficient degree of branching, using suitable micro-organisms or their branching enzymes (EC 2.4.1.18) capable of producing the desired degree of branching. Examples of suitable branching enzymes are α-(1,4)-D-glucan: α(1,4)glucan 6-glycosyltransferases and α-(1,4)-D-glucan: orthophosphate α-glycosyltransferases (EC 2.4.1.1). Also blends of different highly branched α-glucans can be used.

It is also possible to produce the highly branched glucans by *de novo* synthesis from glucose or (phosphorylated) glucose derivatives using a combination of a glucan synthesising enzyme and a branching enzyme, or by synthesis form sucrose using a combination of an amylosucrase and a branching enzyme. As an example, highly branched α(1,4)(1,6) glucans can be obtained from sucrose by the combined action of an amylosucrase and an α-(1,4)-D-glucan: α(1,4)glucan 6-glycosyltransferase optionally expressed in modified crops. A degree of branching up to 35% can be obtained as described in EP-A-1117802 (WO 00/22140, PlantTec).

Glycogen is an abundant endogenic energy storage molecule in higher life forms and particular bacteria and has a degree of branching of about 8-12% (Voet and Voet, Biochemistry, John Wiley & Sons, 1990). Glycogen is synthesised by glucosyl transferases using activated glucose as a substrate, being an economically unattractive process for industrial purposes. Other means to obtain highly branched α(1,4)(1,6) glucans is offered via several enzymatic routes (in planta or in situ with micro-organisms or their enzymes as illustrated in EP-A-1117802.

The highly branched α-glucans obtained by the action of glucosyltransferase on sucrose as described in WO 01/90372 have a molecular weight of at least 10⁷ as determined by SEC-MALLS-RI (Van Geel, I. *et al. Appl. Environ. Microbiol.* (1999) 65, 3008-3014) and a very compact configuration. They are referred to as "reuteran". This compactness is demonstrated by the radius of giration, showing a level of only 35 nm. The degree of branching can exceed 15% for these particular glucans. The thickening effect demonstrated for reuteran as a function of the pH lowering using hydrochloric acid can be explained by the Hofineister effect as reviewed by Collins (Collins, K.D. Washabaugh, M.W., (1985), *Quart. Rev. Biophys.* **18**, 323). The increase in water structure disrupting chloride ion concentration leads to a better water binding by the polysaccharide reuteran. The improved water binding is expected to result in the observed viscosity increase, which is relevant for stomach conditions. In contrast, if water structure improving electrolytes are used like citrate, reuteran will be deprived of its water binding capacity as is demonstrated in the examples below.

The glucans to be used according to the invention should have a reduced digestibility, i.e. increased resistance to degradation by intestinal enzymes, compared to e.g. starch. Preferably, the digestibility (expressed as percentage glucose release in jejunum and ileum e.g. in the TNO gastro-intestinal model for the small intestine: Minekus *et al,* Alternatives to Laboratory Animals (ATLA) 23: 197-209) should be less than 30% compared to native starch. Thus, the glucans have nutritional fibre characteristics.

The highly branched glucans can be used as an ingredient in products used for medical, nutraceutical and food applications, especially liquid products preferably also containing a proteinaceous compound. The glucan can be dosed at a level ranging from 0.5 up to 10 wt.%, preferably from 1 to 8 wt% (w/w), preferably in combination with a food or pharma grade protein present at levels ranging between 0 up to 15% (w/w) on total product.

In a preferred embodiment of the invention highly branched α(1,4)(1,6) glucans are used in liquid products for example a drink or beverage, preferably also containing proteins. More preferably this drink is a flavoured or fermented milk drink such as a yoghurt-like drink. Advantageously, the drink may be inoculated with a lactic acid bacterium, such as *Lactobacillus reuteri,* expressing said glucosyltransferase activity combined with a sucrose substrate. The drink is stabilised and textured by the soluble α(1,4)(1,6)-glucan while at the same time a significant thickening effect is obtained after entering the acidic conditions of the stomach. Other suitable forms of the liquid composition of the invention include fruit drinks, other dairy products, soups, sauces, or specially designed solutions or dispersions. Where reference is made herein to a liquid composition or liquid product, this naturally extends to a non-liquid or semi-liquid product having the same weight ratios of its ingredients, but a different (i.e. lower) solvent content. Thus a liquid product also covers a concentrate or a dry form which after suitable dilution or addition of water of other aqueous solvents and possibly further ingredients produces a liquid composition which is ready for use.

The liquid composition of the invention preferably also contains a protein, which can be any food or pharma grade protein showing a good stability and solubility at pH 3-6; such protein is referred to herein as "food protein". Suitable food proteins include milk proteins such as casein and whey proteins, soy, pea, fungal, egg protein and gelatine, or their hydrolysates. The protein may be incorporated in the liquid composition at a level of e.g. 0.5 - 15 g per 100 g, preferably 2-10 wt.%. The preferred weight ratio between α-glucan and protein is between 0.02 and 10, preferably between 0.2 and 5.

The composition may advantageously also contain further nutritional fibres, e.g. soluble fermentable or non-fermentable fibres such as pectin, gum arabic, inulin, arabinans, galactomannans or oligosaccharides, and/or insoluble fibres such as cellulose, other β-glucans and hemicellulose, e.g. at a level of 0.5-5 g per 100 g. The weight ratio between the poorly digestible branched α-1,6 linkages-containing glucans to be used according to the invention and other nutritional fibres is preferably between 19 and 0.2, more preferably between 9 and 0.5. The composition may further contain fats, fat replacers, other weight management components, emulsifiers, thickeners, stabilisers, flavours, colorants, salts, vitamins, and other food components, such as fruits, nuts, cereals, etc.

The invention also pertains to method of inducing satiety to a person in need thereof, in particular for weight management purposes or for preventing or curing obesity, by administering the branched α-glucan described above, preferably in the presence of proteins, typically in the form of a drink containing the α-glucan at a level of e.g. 1-10% by weight. The dosage can be adapted to the required satiety and to further individual needs and conditions of the subject. For example, an amount from 1 g per day can be administered up to e.g. 250 g per day, in a single dosage, or preferably divided over 2-4 daily dosages. Preferred amounts are between 5 and 50 g of α-glucan per day. Advantageously, the glucan is administered on a regular, repeated basis, e.g. for at least 7 days or more.

Advantages of the present invention are created by improved satiety feeling for its consumers, better product appearance, lower caloric content, lower formulation costs also due to little legislative barriers and a clean labelling for the target food products using the ingredient.

Additional advantages in using these compounds for the application targeted are found in the fact that no heat treatment needs to be applied in order to functionalise the ingredient like for granular amylopectin starch and no retrogradation phenomena are observed in time leading to an improved stability. Also, the detrimental effects of human endogenous amylase activity on the thickening effect are not observed for reuteran, which also leads to a low glycemic index.

*In vitro* experiments have demonstrated that this pH-triggered thickening effect results in an instantaneous increase of the viscosity. This viscosity effect creates opportunities for product developers to incorporate other active components in the drink which need a retained release from the stomach into the ileum.

Other advantages provided by the highly branched α-glucan is the limited degradation during passage through the small intestine lowering the glycemic index of the product. This makes it also a very suitable ingredient for formulations used by diabetics.

Other embodiments of the invention are the use of highly branched α-glucan in the formulation of solid or semi-solid products creating also a viscosifying effect in the stomach. Also applications in feed or pet food are part of the invention.

### Example 1. Production of reuteran

Strain Lb 35-5 was produced as described in WO 01/90372 and subsequently concentrated and washed by centrifugation using an 0.1 M acetic acid buffer. The cells were anaerobically suspended in a 150g/l sucrose 0.1 M acetic acid buffer pH 5.5 overnight under stirring. The following day the cells were removed by centrifugation and the reuteran was washed using diafiltration in a 150 kDa ultrafiltration unit, until unreacted sucrose could not be detected anymore. The retentate was freeze-dried and the resulting reuteran was used for further experiments.

### Example 2. Physical properties in water and milk drink

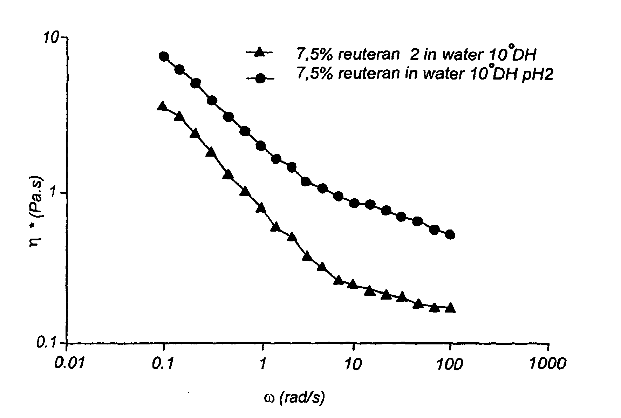

Reuteran obtained as described according to example 1 was added at a 5 wt.% level to a commercial milk drink with a pH of 4 (Friesland Coberco Dairy Foods: Fristy no sugar added, multi-fruit flavour). The original milk drink and the milk drink with reuteran were evaluated for their viscosity as a function of time and concentration of HCl. For this purpose the viscosity of the drinks was measured using a Rheometrics RFSII dynamic rheometer with a Couette DIN geometry at a temperature of 37°. As a reference also a 7.5 wt% reuteran solution in water with a calcium level of 0.2621 g/l CaCl₂·2H₂O was made with a pH of 6.8. Each sample either was compensated for the volume of 4 M HCl added to the acidified samples. At first a strain sweep was executed between 0.1-100% at 10 rad/s for the milk drink in order to select a deformation in the linear region for the time sweep at 10 rad/s (for the acidified samples) or the frequency sweep between 0.1-100 rad/s (for the original solutions). The measurements were performed directly after sample preparation.
Table 1 shows the thickening effect for the original milk drink as well as for the milk drink containing 5 wt% reuteran as a function of change in HCl concentration and time. The thickening effect triggered by the drop in pH is drastically amplified by reuteran. The milk drink containing reuteran demonstrates a very useful thickening effect due to increased HCl concentration at a physiologically relevant level. Also the pure reuteran solution in water shows a clear thickening effect due to an increase in HCl concentration as can be derived from the frequency sweeps collected in figure 1 (Frequency sweep for a 7.5wt% reuteran solution in water (10° DH) at two different pH values). The resulting viscosities at 10 rad/s are 0.24 at pH 6.8 and 0.86 after pH drop (pH 2).

**Table 1: Viscosity (Pa.s) of milk drink at 10 rad/s**

| | pH 4 | pH 2, 0 h | pH 2, 1 h | pH 2, 2 h | pH 2, 3 h |
|---|---|---|---|---|---|
| milk drink | 0.01 | 0.01 | 0.02 | 0.025 | 0.03 |
| milk drink with 5% reuteran | 0.25 | 0.37 | 0.75 | 0.84 | 0.88 |

### Example 3. Thickening effect as a function of the degree of branching

In order to demonstrate the importance of the degree of branching the following experiment was designed. Two branched α-glucans were selected being reuteran with a level of α(1,4,6) linked AGU of 16% (Mw 3.5·10⁶) (van Geel I. et.al., *Appl. Environ. Microbiol.(1999),* 65, 3008-3014) and glycogen obtained from Merck (order nr. 4202) with a level of α(1,4,6) linked AGU of 8% (Mw 0.27-3.5·10⁶). As a control a pullulan (non-branched) containing 31% α(1,6) linked AGU (Mw 3.5·10⁶) was also tested.
The three polysaccharides were dosed in the milk drink as used in example 2 at a 5wt% level. For the three milk drinks and a reference a frequency sweep was executed as described in example 2. The viscosity at 10 rad/s for the non-acidified drinks was derived from a separate frequency sweep as described before.
The results for these measurements are collected in table 2 showing the viscosity at 10 rad/s as these were derived from the frequency sweeps executed for the acidified and non-acidified drinks, respectively. The α-glucans containing α(1,4,6) linked AGU show a clear pH triggered thickening effect. In contrast, the pullulan not having α(1,4,6) linked AGU's does not demonstrate a thickening effect in the milk drink upon pH lowering.

**Table 2: Viscosity (Pa.s) of milk drink with different glucans**

| | Viscosity (Pa·s) | | Viscosity increase |
|---|---|---|---|
| | pH4 | pH2 | |
| 5 wt% reuteran in milk drink | 0.2553 | 0.3695 | 45% |
| 5 wt% glycogen in milk drink | 0.0064 | 0.0411 | 540 % |
| 5 wt% pullulan in milk drink | 0.8051 | 0.3661 | . -55 % |

### Example 4

Different amounts of reuteran (0, 1, 2 and 5% w/w) were added to Slim-Fast (commercially available) and thermostated in a water bath at 37 °C. The pH of the samples was adjusted to pH 2 by adding 4M HCl. For the controls (no pH lowering), an equal amount of water (10 DH) was added. The rheology of the samples was measured using a Rheometrics RFS II with Couette DIN geometry at 37 °C. After thermostating, a strain sweep was performed at 37°C. The pH-dependent thickening effects are summarised in table 3 showing the critical G* values.

**Table 3: G values (Pa) Slim-Fast with different amounts of retiteran**

| G* critical (Pa) | G* (Pa) |
|---|---|
| 5% reuteran in Slim-Fast | 8.0 |
| Slim-Fast pH2 control | 15.5 |
| 1% reuteran in Slim-Fast pH2 | 20.0 |
| 2% reuteran in Slim-Fast pH2 | 29.5 |
| 5% reuteran in Slim-Fast pH2 | 47.0 |

## Claims

1. Use of a branched α-glucan having an average molar weight of at least 10⁵ Da and having a degree of branching of at least 8%, for preparing a liquid nutritional or pharmaceutical composition for inducing or enhancing satiety and/or satiation.

2. Use according to claim 1, wherein the α-glucan has a degree of branching of at least 10%, preferably at least 12%.

3. Use according to claim 1 or 2, wherein the α-glucan has an average molar weight of between 5·10⁵ and 10⁸ Da.

4. Use according to any one of claims 1-3, wherein the α-glucan contains α(1,4) and α(1,6) linkages.

5. Use according to any one of claims 1-4, wherein the α-glucan is non-ionic.

6. Use according to any one of claims 1-5, wherein the α-glucan is produced by enzymatic glucosyl transfer from sucrose.

7. Use according to any one of claims 1-6, wherein the α-glucan is used in a concentration of 1-10 % (by weight).

8. Use according to any one of claims 1-7, wherein the α-glucan is combined with a protein, especially a processed milk or soy protein.

9. Use according to any one of claims 1-8, wherein an aqueous solution of 7.5 wt.% the α-glucan at pH 2 shows an increase in viscosity of at least 1.5 times compared to the viscosity at pH 6.8, measured at 10 rad/s.

10. Liquid food composition, containing 1-10 wt.% of an α-glucan having an average molar weight of at least 10⁵ Da and having a degree of branching of at least 8%, and at least 1 wt.% of a food protein.

11. A method of inducing satiety and satiation in a person in need thereof, comprising repeatedly administering to that person an effective amount of a branched α-glucan having an average molar weight of at least 10⁵ Da.

## Revendications

1. Utilisation d'un α-glucane ramifié ayant une masse molaire moyenne d'au moins 10⁵ Da et un degré de ramification d'au moins 8%, pour préparer une composition nutritionnelle ou pharmaceutique liquide destinée à induire ou à accroître la satiété et/ou le rassasiement.

2. Utilisation selon la revendication 1, dans laquelle l'α-glucane a un degré de ramification d'au moins 10%, de préférence d'au moins 12%.

3. Utilisation selon la revendication 1 ou 2, dans laquelle l'α-glucane a une masse molaire moyenne comprise entre 5.10⁵ et 10⁸ Da.

4. Utilisation selon l'une quelconque des revendications 1 à 3, dans laquelle l'α-glucane contient des liaisons α(1,4) et α(1,6).

5. Utilisation selon l'une quelconque des revendications 1 à 4, dans laquelle l'α-glucane est non ionique.

6. Utilisation selon l'une quelconque des revendications 1 à 5, dans laquelle l'α-glucane est produit par transfert enzymatique de glucosyle à partir du saccharose.

7. Utilisation selon l'une quelconque des revendications 1 à 6, dans laquelle l'α-glucane est utilisé à une concentration de 1 à 10% (en poids).

8. Utilisation selon l'une quelconque des revendications 1 à 7, dans laquelle l'α-glucane est combiné avec une protéine, en particulier une protéine de lait ou de soja transformé.

9. Utilisation selon l'une quelconque des revendications 1 à 8, dans laquelle une solution aqueuse de 7,5% en poids de l'α-glucane à pH 2 présente une augmentation de viscosité d'au moins 1,5 fois par rapport à la viscosité à pH 6,8, mesurée à 10 rad/s.

10. Composition alimentaire liquide contenant 1 à 10% en poids d'un α-glucane ayant une masse molaire moyenne d'au moins 10⁵ Da et un degré de ramification d'au moins 8%, et au moins 1% en poids d'une protéine alimentaire.

11. Procédé d'induction de la satiété et du rassasiement chez une personne qui en a besoin, comprenant l'administration de manière répétée à cette personne d'une quantité efficace d'un α-glucane ramifié ayant une masse molaire moyenne d'au moins 10⁵ Da.

## Patentansprüche

1. Verwendung eines verzweigten α-Glucans, mit einem durchschnittlichen Molgewicht von mindestens 10⁵ Da und einem Verzweigungsgrad von mindestens 8%, zum Herstellen einer flüssigen Nähr- oder pharmazeutischen Zusammensetzung, um Sattheit und/oder Sättigung hervorzurufen oder zu verstärken.

2. Verwendung nach Anspruch 1, wobei das α-Glucan einen Verzweigungsgrad von mindestens 10%, vorzugsweise mindestens 12% hat.

3. Verwendung nach Anspruch 1 oder 2, wobei das α-Glucan ein durchschnittliches Molgewicht von zwischen 5·10⁵ und 10⁸ Da besitzt.

4. Verwendung nach einem beliebigen der Ansprüche 1-3, wobei das α-Glucan α(1,4)-und α(1,6)-Verknüpfungen enthält.

5. Verwendung nach einem beliebigen der Ansprüche 1-4, wobei das α-Glucan nichtionisch ist.

6. Verwendung nach einem beliebigen der Ansprüche 1-5, wobei das α-Glucan durch enzymatischen Glucosyltransfer aus Saccharose hergestellt wird.

7. Verwendung nach einem beliebigen der Ansprüche 1-6, wobei das α-Glucan in einer Konzentration von 1-10% (Gewichtsprozent) verwendet wird.

8. Verwendung nach einem beliebigen der Ansprüche 1-7, wobei das α-Glucan mit einem Protein kombiniert wird, besonders einem verarbeiteten Milch- oder SojaProtein.

9. Verwendung nach einem beliebigen der Ansprüche 1-8, wobei eine wässrige Lösung von 7,5 Gewichtsprozent des α-Glucans bei pH 2 einen Anstieg an Viskosität von mindestens dem 1,5-fachen, verglichen mit der Viskosität bei pH 6,8, zeigt, gemessen bei 10 rad/s.

10. Flüssige Nahrungsmittelzusammensetzung, enthaltend 1-10 Gewichtsprozent eines α-Glucans, mit einem durchschnittlichen Molgewicht von mindestens 10⁵ Da und einem Verzweigungsgrad von mindestens 8% und mindestens 1 Gewichtsprozent eines Nahrungsmittelproteins.

11. Verfahren zum Hervorrufen von Sattheit und Sättigung in einer Person mit Bedarf daran, umfassend das wiederholte Verabreichen einer wirksamen Menge eines verzweigten α-Glucans, das ein durchschnittliches Molgewicht von mindestens 10⁵ Da besitzt, an diese Person.
